# EUROPEAN PATENT APPLICATION

(11) **EP 4 632 749 A1**
(43) Date of publication of application: **15.10.2025**
(21) Application number: 25168419.7
(22) Date of filing: 04.04.2025
(51) Int. Cl.: G16C 20/30

(54) **TRAINING DATA FOR MATERIAL PROPERTY PREDICTION**

(30) Priority: 10.04.2024 DE 102024110050
(71) Applicant: Bundesdruckerei GmbH, 10969 Berlin (DE)
(72) Inventor: Kowalski, Hagen-Henrik, 12307 Berlin (DE); Eble, Holger, 10965 Berlin (DE); Simon, Lars, 10117 Berlin (DE); Radons, Manuel, 15712 Königs Wusterhausen (DE); Muth, Oliver, 12277 Berlin (DE)
(74) Representative: Richardt Patentanwälte PartG mbB

(57) **Abstract**

Disclosed is a method of generating a training dataset for training a machine learning model for prediction of a specific property of a material. The method comprises: repeatedly performing the following: selecting a hypothetical material from a parameter space, the parameter space being defined by a set of material parameters descriptive of materials; using a quantum computer for numerically solving an electronic Schrödinger equation for a quantum system representing the selected material; using the solution of the electronic Schrödinger equation for obtaining the specific property of the selected material; adding an entry to the training dataset, the entry indicating the selected material and a label, wherein the label indicates the obtained specific property.

## Description

### FIELD OF THE INVENTION

The disclosure relates to a method for generating a training dataset for training a machine learning model for prediction of a specific property of a material.

### BACKGROUND

The prediction of the color properties of materials may be based on resource-intensive calculations of the electronic structure of the material using density functional theory (DFT). The electronic structure may be exactly represented by a density functional; however, that density functional may not be exactly known and thus may be approximated. The approximate DFT implementation may approximate the full solution to the electronic Schrödinger Equation which itself may have no known efficient classical algorithm to find its solution.

### SUMMARY

Example embodiments provide a method of generating a training dataset for training a machine learning model for prediction of a specific property of a material, the method comprising: repeatedly performing the following: selecting a hypothetical material from a parameter space, the parameter space being defined by a set of material parameters descriptive of materials; using a quantum computer for numerically solving an electronic Schrödinger equation for a quantum system representing the selected material; using the solution of the electronic Schrödinger equation for obtaining the specific property of the selected material; adding an entry to the training dataset, the entry indicating the selected material and a label, wherein the label indicates the obtained specific property.

Example embodiments provide a computer program comprising machine executable instructions, wherein execution of the machine executable instructions causes a computer system to perform at least the following: repeatedly performing the following: selecting a hypothetical material from a parameter space, the parameter space being defined by material parameters descriptive of materials; using a quantum computer for numerically solving an electronic Schrödinger equation for a quantum system representing the selected material; using the solution of the electronic Schrödinger equation for obtaining the specific property of the selected material; adding an entry to the training dataset, the entry indicating the selected material and a label, wherein the label indicates the obtained specific property.

Example embodiments provide a computer system of generating a training dataset for training a machine learning model for prediction of a specific property of a material. The computer system is configured for: repeatedly performing the following: selecting a hypothetical material from a parameter space, the parameter space being defined by material parameters descriptive of materials; using a quantum computer for numerically solving an electronic Schrödinger equation for a quantum system representing the selected material; using the solution of the electronic Schrödinger equation for obtaining the specific property of the selected material; adding an entry to the training dataset, the entry indicating the selected material and a label, wherein the label indicates the obtained specific property.

### BRIEF DESCRIPTION OF THE DRAWINGS

In the following, examples are described in greater detail making reference to the drawings in which:
Fig. 1 is a diagram illustrating a computer system in accordance with an example of the present subject matter.
Fig. 2 is a flowchart of a method of generating a training dataset for training a machine learning model for material property prediction in accordance with an example of the present subject matter.
Fig. 3 is a diagram of a data structure representing the training dataset generated in accordance with an example of the present subject matter.
Fig. 4 is a flowchart of a method of training a machine learning model for material property prediction in accordance with an example of the present subject matter.
Fig. 5 is a flowchart of using a trained machine learning model for material property prediction in accordance with an example of the present subject matter.
Fig. 6 is a diagram illustrating different phases for enabling prediction of specific properties of materials in accordance with an example of the present subject matter.
Fig. 7 is a block diagram of an exemplary computer system for implementing at least part of the present method in accordance with an example of the present subject matter.

### DETAILED DESCRIPTION

In the following description, for purposes of explanation and not limitation, specific details are set forth such as particular architectures, interfaces, techniques, etc., in order to provide a thorough understanding of the examples. However, it will be apparent to those skilled in the art that the disclosed subject matter may be practiced in other illustrative examples that depart from these specific details. In some instances, detailed descriptions of well-known devices and/or methods are omitted so as not to obscure the description with unnecessary detail.

Identity (ID) document may need to be secured against counterfeiting. For that, different security features may be used in combination in the ID document. The security features may include holograms, special inks, watermarks, security threads or other visual and physical elements. The present subject matter may enable the development of new (polymer) colors for security features in ID documents by accurately predicting specific properties of materials, wherein said property may be used for the development of the new colors for the security features. For example, machine learning models may be used to predict the specific properties of materials. The present subject matter may provide optimal training data for training these models. The resulting trained models may have a higher prediction accuracy of material properties.

For that, a training dataset may be generated in order to train a machine learning model to predict a specific property of materials. The training dataset may be created using a set of hypothetical materials. The hypothetical material may be a material. The hypothetical material may be a material that could exist, but not have been made and/or characterized experimentally. Each hypothetical material of the set of hypothetical materials may be selected from the parameter space. The parameter space may comprise hypothetical materials that can be selected, wherein each hypothetical material may have a hypothetical composition and a crystal structure. The parameter space may be defined by the set of material parameters which are descriptive of the materials. The set of parameters of the material may provide a representation of the material and capture precise details of atomic configurations within the material. The set of parameters of the material may, for example, be descriptive of an atomic structure of the material and/or crystal structure of the material. The hypothetical material may, for example, be a polymer. The polymer may be any natural or synthetic material e.g., composed of large molecules. The parameter of the material may, for example, be anyone of: nuclear charge or orbital radius. The parameter space may thus propose hypothetical compounds enabling new useful functional materials. Each selected hypothetical material may represent a point in the parameter space having specific values of the set of parameters which are allowed by the parameter space. For example, the points in the parameter space may be defined using existing materials. The parameter space may enable the design of materials. In one example, the material may be selected such that the corresponding electronic Schrödinger equation may be solved numerically on the quantum computer. In one example, the selected material may have a number of atoms smaller than a threshold. The quantum computer may utilize the principles of quantum mechanics to perform quantum computations by operating qubits.

The quantum system may be defined for each selected hypothetical material. This may result in a set of quantum systems. The quantum computer may be used for numerically solving the electronic Schrödinger equation for each quantum system representing the respective hypothetical material. The electronic Schrödinger equation may, for example, be a time-independent electronic Schrödinger equation. The electronic Schrödinger equation may, for example, be obtained using the Born-Oppenheimer approximation. The Born-Oppenheimer approximation may enable to separate the time-independent Schrödinger equation of the quantum system into the electronic Schrödinger equation and the nuclear Schrödinger equation. The numerical solution of the electronic Schrödinger equation may be used for obtaining the specific property of each hypothetical material. The numerical solution of the electronic Schrödinger equation may provide the electronic structure of the material. The electronic structure may be used to determine the specific property of the material. This may result in a set of values of the specific property for the set of hypothetical materials respectively. For each hypothetical material of the set of hypothetical materials, an entry may be included in the training dataset. The entry may comprise a description of the hypothetical material and a label indicating the obtained specific property of the hypothetical material. The description of the material may, for example, comprise values of the set of parameters.

For example, each entry of the training dataset may comprise a tuple (*Mᵢ*, *labelᵢ*), where *Mᵢ* is the description of the *i*-th hypothetical material of the set of hypothetical materials and the *labelᵢ* is the label of the *i*-th hypothetical material. In one example, the training dataset may be stored in a storage system of the computer system. The computer system may control access to the training dataset. For example, the computer system may define permissions, such as read permission and execute permissions, for access to the training dataset. One or more users may use the training dataset based on permissions which are assigned to the users.

The electronic Schrödinger equation may, for example, be solved (e.g., with linear scaling) on the quantum computer using Full Configuration Interaction.

The numerical solution of the electronic Schrödinger equation may contain the material properties to determine the material color. Indeed, the solution may provide the wave function which may constitute the most complete description of the quantum system. An observable may be provided for observing or measuring the specific property of the material. The observable may be represented by an operator. If the wavefunction is an eigenstate of the operator, then the value measured for the observable may be the eigenvalue. If the wavefunction is not an eigenstate of the operator, the expectation value may be used to determine the specific property. In one example, the eigenstates of the operator may form a complete set such that the wave function may be written as a linear combination of these eigenstates, and thus enabling the evaluation of the specific property.

The electronic Schrödinger equation may be defined by a Hamiltonian of the quantum system. The band gap may, for example, be the energy difference (e.g., in electron volts) between the top of the valence band and the bottom of the conduction band. The band gap (BG) of the material may, for example, be a difference between the electron affinity (EA) of the material and the ionisation potential (IP) of the material, BG = IP - EA. The ionisation potential may be the energy required to supply to a valence electron of the material in order to extract that electron. The electron affinity may be the amount of energy released when the material gains an electron.

The determination of the electron affinity of the material and the ionization potential of the material may be performed by solving the electronic Schrodinger Equation for the ground state and for the excited states associated with the ionisation potential and the electron affinity respectively. In one example, the variational quantum eigensolver (VQE) may be used to determine the electron affinity and the ionisation potential.

For example, the Born-Oppenheimer approximation may be used to obtain the electronic Schrödinger equation. According to the Born-Oppenheimer approximation, the kinetic energy of the nuclei can be neglected since it may be smaller than the kinetic energy of the electrons, the electrons may then be considered as moving in a field of fixed nuclei, where the nuclear-nuclear repulsion may be a constant. The solution of the electronic Schrödinger equation may be the electronic wavefunction which describes the electrons, e.g., it describes the motion of the electrons. The electronic Schrödinger equation associated with the material may be solved using the Hartree-Fock (HF) method and the VQE algorithm in order to obtain the ground-state wavefunction. In the VQE algorithm, a unitary evolution operator may be implemented on the quantum computer using a parameterized quantum circuit. The parameters of the quantum circuit may be optimized to variationally minimize the material energy and obtain the material ground state. In order to use the VQE algorithm, a suitable initial state of the quantum circuit may be used. The initial state may be obtained using the Hartree-Fock method. The VQE algorithm may be used to find a wave function of the material and estimate the expectation value of its Hamiltonian while a classical computer may be used to adjust the quantum circuit parameters in order to find the material's ground state energy. For example, by minimizing the expected value, the minimum eigenvalue of the Hamiltonian may be obtained, and then the solution to the molecular ground state energy may be completed.

The ground-state wave function may be used to obtain the excited states associated with the ionization potential and with the electron affinity respectively. This may be performed using for example the method described in the papers: Ayush Asthana et al. Chem. Sci., 2023, 14, 2405 or Kenji Sugisaki et al. J. Phys. Chem. Lett. 2021, 12, 2880-2885. The band gap may thus be obtained by computing the difference between the ionization potential and the electron affinity.

According to one example, the specific property of the hypothetical material is a band gap. That is, the label in each entry of the training dataset may comprise a value of the band gap of the hypothetical material associated with the entry. This may be advantageous as the training dataset may, for example, be large enough for accurate color predictions using band gaps. The band gap may refer to the energy difference between the conduction band and the valence band. For example, the band gap may be the energy difference (e.g., in electron volts) between the top of the valence band and the bottom of the conduction band. The band gap of the material may determine its color.

According to one example, the parameter space comprises points, each point having respective values of the set of material parameters, wherein the parameters space is defined using a bin packing problem such that the parameter space is uniformly filled by the points. The bin packing problem may enable an optimization problem, in which items of different sizes may be packed into a finite number of bins or containers, each of a fixed given capacity, in a way that minimizes the number of bins used. This example may enable to control the size as well as the structure of the parameter space.

According to one example, the solution of the electronic Schrödinger equation comprises a wave function that defines a Hilbert space, wherein the specific property is obtained by providing an observable having specific basis in the Hilbert space and by projecting the wave function into the specific basis.

It is known that the Schrödinger equation may be solved by a quantum computer e.g., using the Full Configuration Interaction. The Quantum computer may provide computational tools for accurately calculating state properties of many body systems. A large training dataset of exact color predictions may thus be generated through the numerical solution of the electronic Schrödinger equation.

According to one example, the parameter space is defined by Smooth Overlap of Atomic Positions (SOAP) descriptors. The SOAP descriptor may characterize the material. The descriptor of the material may, for example, be a tuple of real valued functions of the atomic positions, e.g., bond lengths, bond angles, etc. of the material. The SOAP descriptors may provide appropriate representation of material structures. This may further improve the accuracy of the predictions of the trained model because the model is trained based on accurate representations of the hypothetical materials. Another advantage may be that the level of details of the descriptors may be chosen for enabling coarser or finer fingerprinting. Coarser fingerprinting may save resources while providing reliable results especially as the required accuracy for the property prediction is less critical. If the accuracy in property predictions is high, fingerprinting may be finer. The descriptor may, for example, be provided as a vector whose entries represent the set of parameters respectively.

According to one example, the set of parameters are descriptive of at least one of chemical composition of the material or atomic configuration of the material. The set of material parameters may, for example, comprise: nuclear charge, orbital radius electronegativity and ionization potentials.

According to one example, the method further comprises using the training dataset to train the machine learning model to predict the specific property for input materials. For example, based on the training dataset, a classical machine learning model is trained using as input the chemical composition, the structure as well as the atomic properties of the different elements of the material in order to predict the band gap and thus the color properties of the material. By using accurately determined band gaps to train the machine learning model, the predictions may be similarly at high accuracy level. The model may make accurate predictions without the use of a quantum computer. By contrast to DFT-based calculations of the electronic structure of a material which may typically give significantly too small (Local Density Approximation) or too large (Generalized Gradient Approximation) predictions of the band gap, the present subject matter may provide more accurate predictions of the band gap.

According to one example, a description of a desired material may be received. The desired material may, for example, be selected from the parameter space. The description of the desired material may be input to the trained machine learning model. An output of the trained machine learning model may be received. The output indicates the band gap of the desired material. In another example, a description of each desired material of a set of two or more desired materials may be received. The set of desired materials may, for example, be selected from the parameter space. The description of the each desired material may be input to the trained machine learning model. An output of the trained machine learning model may be received. The output indicates the band gap of the each desired material. In case the one or more desired materials have the desired colors, the description of the one or more desired material may be input into a robotic system for providing the identity document. The robotic system may include automated chemical reactors, and/or material deposition techniques under precise control over temperature, pressure, and composition.

For example, in case one or more of the desired materials have the desired one or more colors respectively, the description of the one or more desired materials may be input into a robotic system. The robotic system may include automated chemical reactors and/or a system that is configured to implement material deposition techniques. The robot system may, for example, be used for manufacturing or surface coating of identity documents using the one or more desired materials.

The machine learning model may, for example, be a neural network or deep neural network such as convolutional neural network (CNN). The machine learning model that may quickly make predictions about material colors even on classical computers. The present approach may be a hybrid approach because it uses the quantum computer to generate training dataset and then use the trained model in a classical computer. The precision achieved with the machine learning model may not be achieved with purely classical methods (i.e., without using a quantum computer).

According to one example, in case the specific property is the band gap, the method further comprises: predicting the band gap of a hypothetical material using the trained machine learning model; and in response to determining that a color determined by the band gap fulfils a selection criterion, using the hypothetical material for creating an identity document. The selection criterion may require that the band gap, that determined the color, is in a specific range. This may enable to provide high secure ID documents using the material having the selected colors.

According to one example, in case the specific property is the band gap, the method further comprises: predicting the band gap of one or more hypothetical materials using the trained machine learning model; and in response to determining that a color determined by the one or more band gaps is user desired color, using the one or more hypothetical materials for creating an identity document. The one or more hypothetical materials may, for example, be selected from the parameter space. The one or more hypothetical material may be used for providing color to the identity document.

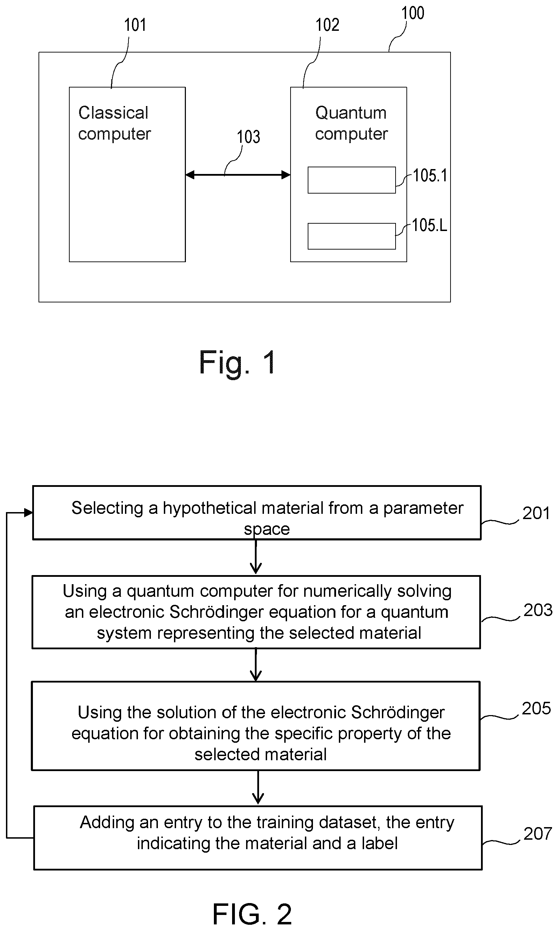

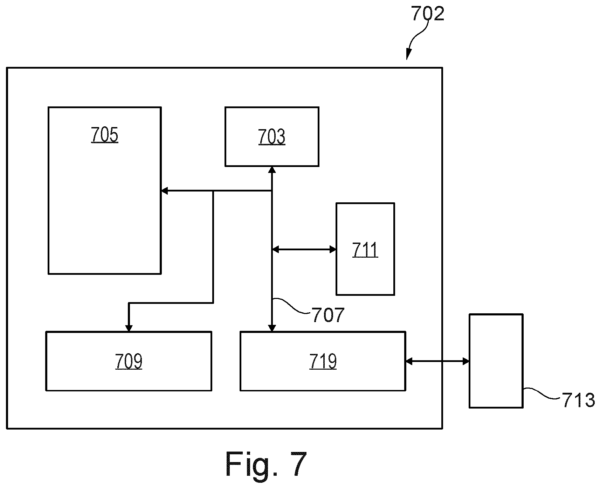

**Fig. 1** is a diagram illustrating a computer system in accordance with an example of the present subject matter. The computer system 100 comprises a classical computer 101. The computer system 100 may further comprise a quantum computer 102. An example implementation of the classical computer 101 is described with reference to Fig. 7. The quantum computer 102 may comprise qubits. For example, qubits may be part of quantum registers 105.1 through 105.L. The classical computer 101 may be configured to control operation of the quantum computer 102. The classical computer 101 may use an interface 103 with the quantum computer 102 to control operation of the quantum computer 102 in accordance with an example of the present subject matter.

**Fig. 2** is a flowchart of a method of generating a training dataset for training a machine learning model for prediction of a specific property of a material in accordance with an example of the present subject matter. For the purpose of explanation, the method described in Fig. 2 may be implemented in the system illustrated in Fig. 1, but is not limited to this implementation. For example, the method of Fig. 2 may be performed by the computer system 100.

A hypothetical material may be selected in step 201 from a parameter space. The parameter space is defined by a set of material parameters descriptive of materials.

A quantum computer (e.g., 102) may be used in step 203 for numerically solving an electronic Schrödinger equation for a quantum system representing the selected hypothetical material. For example, the classical computer 101 may control the quantum computer 102 in order to numerically solve the electronic Schrödinger equation for the quantum system representing the selected hypothetical material.

The solution of the electronic Schrödinger equation may be used for obtaining in step 205 the specific property of the selected hypothetical material.

An entry may be added in step 207 to the training dataset. The entry indicates the selected material and a label, wherein the label indicates the obtained specific property. The entry may comprise a description of the material and the label which is property of the material.

As indicated in Fig. 2, steps 201 to 207 may be repeated for each further selected hypothetical material. The repetition may be performed until a stopping criterion is fulfilled. The stopping criterion may, for example, require that a maximum number of repetitions is reached or that a number of entries of the training dataset is reached.

In one example implementation of Fig. 2, the (whole) method may be repeated e.g., on a periodic basis, wherein the training dataset is updated/increased with new entries obtained for each further execution of the method.

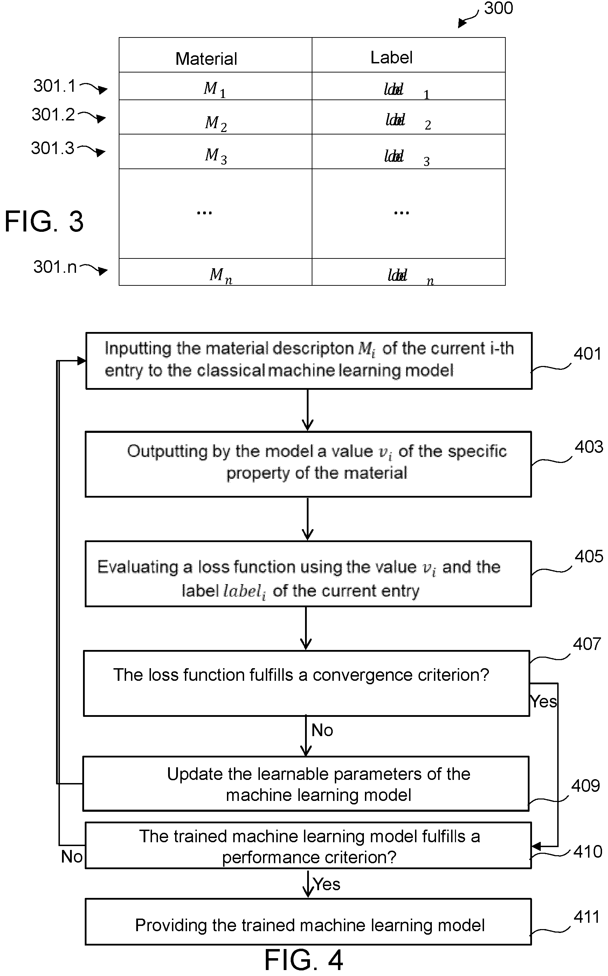

**Fig. 3** is a diagram of a data structure representing the training dataset generated in accordance with an example of the present subject matter. The data structure 300 comprises n entries 301.1 through 301.n. Each i-th entry 301.i of the data structure comprises a tuple (*Mᵢ, labelᵢ*), where *Mᵢ* is a description of the i-th material and the *labelᵢ* is the label of the i-th material.

**Fig. 4** is a flowchart of a method for training a classical machine learning model in accordance with an example of the present subject matter. The training may be performed using the training dataset as generated e.g., by the method of Fig. 2. For simplification, Fig. 4 may be described with reference to the data structure of Fig. 3. The entries 301.1-n may be processed one by one using the method. The classical machine learning model may, for example, be a deep neural network such as a CNN or a support vector machine.

In step 401, the description *Mᵢ* of the current i-th entry 301.i may be input to the classical machine learning model. In response, the machine learning model may ouput in step 403 a value *vᵢ* of the specific property (e.g., band gap) of the material *Mᵢ.* A loss function may be evaluated in step 405 using the value *vᵢ* and the label *labelᵢ* of the current entry 301.i. In case (407) the loss function does not fulfill a convergence criterion, the leanrable weights of the machine learning model may be updated in step 409 and steps 401 to 407 may be repeated for a next entry of the training dataset 300. The update of the learnable weights may be performed using gradient descent. For example, the convergence criterion may be defined using a numerical toelrance value. The convergence criterion may, for exmaple, be fulfilled if the difference of the loss function between two iterations is less than that tolerance value. If this tolerance value can't be achieved, there may be a warning and the training may be repeated with a larger tolerance value. For exmaple, the tolerance value may be changed if the training takes too long or if the results are not accurate enough e.g., the change may be performed by choosing a looser criterion that may lead to a shorter training time. In case the loss function fulfills the convergence criterion, it may be determined in step 410 whether the trained machine learning model fulfils a performance criterion. The performance criterion may be defined in accordance with a cross-validation. The cross-validation may be used to evaluate the performance of the trained model on a validation dataset. The validation dataset may, for example, comprise unseen data. The performance criterion may require the trained machine learning model surpasses predefined thresholds for one or more performance evaluation metrics. In case the trained machine learning model fulfils the performance criterion, the trained machine learning model may be provided in step 411. In case the trained machine learning model does not fulfil the performance criterion, the method may be repeated by going back to step 401 for retraining the machine learning model. The repetition may be performed using a different training dataset for training and a different validation dataset for the validation in step 410. The repetition may be performed until the trained machine learning model is validated. The validation may ensure that the model generalizes to data it has not been trained on. This may enable to avoid overfitting or underfitting.

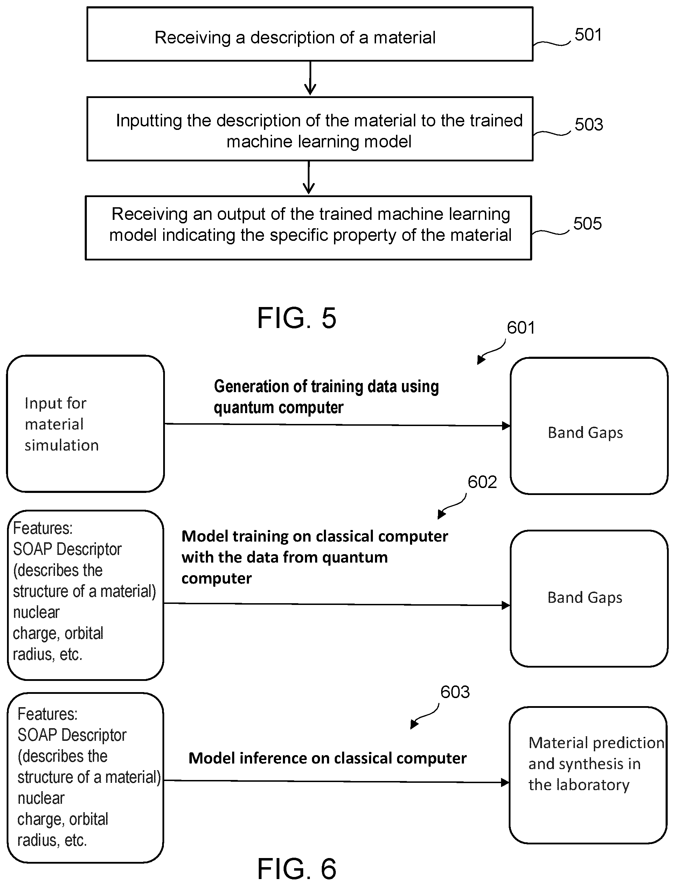

**Fig. 5** is a flowchart of a method for material's band gap prediction in accordance with an example of the present subject matter.

A material description may be received in step 501. The material description may be input in step 503 to a trained machine learning model (e.g., which is obtained in Fig. 4). An output of the trained machine learning model may be received in step 505 from the trained machine learning model. The output indicates the band gap of the input material.

The method of Fig. 5 may, for example, be performed by the computer system of Fig. 7.

**Fig. 6** is a diagram illustrating different phases for enabling prediction of specific properties of materials in accordance with an example of the present subject matter. The phases include a first phase 601 for generation of trainng dataset using a quantum computer. The first phase 601 uses as input hypetetical materials in order to determine the specific property of the input materials, wherein the specific property may be the band gap. The phases include a second phase 602 for training a machine learnining model in a classical computer system using the training dataset obtained in the first phase 601. The machine learning model may use as input the description of the materials of the training dataset in order to be trained to predict the band gap of the materials. The description of the material may comprise features which are obtained by the SOAP descriptor. The phases include a third phase 603 for using or inference of the trained machine learning model which is obtained from the second phase 602. The trained machine learning model may receive as input the SOAP descriptor of the material and may provided as output a prediction of the specific property scuh as the band gap.

**Fig. 7** is a block diagram of an exemplary computer system for implementing at least part of the present method in accordance with an example of the present subject matter.

The components of the computer system 702 may include, but are not limited to, one or more processors or processing units 703, a storage system 711, a memory unit 705, and a bus 707 that couples various system components including memory unit 705 to processor 703. The storage system 711 may include for example a hard disk drive (HDD). The memory unit 705 may include computer system readable media in the form of volatile memory, such as random access memory (RAM) and/or cache memory.

The computer system 702 may also communicate with one or more external devices such as a keyboard, a pointing device, a display 713, etc.; one or more devices that enable a user to interact with computer system 702; and/or any devices (e.g., network card, modem, etc.) that enable the computer system 702 to communicate with one or more other computing devices. Such communication can occur via I/O interface(s) 719. Still yet, the computer system 702 can communicate with one or more networks such as a local area network (LAN), a general wide area network (WAN), and/or a public network (e.g., the Internet) via a network adapter 709. As depicted, the network adapter 709 communicates with the other components of the computer system 702 via bus 707.

The memory unit 705 is configured to store applications that are executable on the processor 703. For example, the memory unit 705 may comprise an operating system as well as one or more application programs. The application programs comprise instructions that when executed enable to perform the method described with reference to Fig. 2, 4, or 5.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

The term "computer system" refers to data processing hardware and encompasses all kinds of apparatus, devices, and machines for processing data, including by way of example, a programmable processor, a computer, or multiple processors or computers. The apparatus can also be or further include special purpose logic circuitry, e.g., a central processing unit (CPU), a FPGA (field programmable gate array), or an ASIC (application specific integrated circuit). In some implementations, the data pro-cessing apparatus and/or special purpose logic circuitry may be hardware-based and/or software-based. The apparatus can optionally include code that creates an execution environment for computer programs, e.g., code that constitutes processor firmware, a protocol stack, a database management system, an operating system, or a combination of one or more of them. The present disclosure contemplates the use of data processing apparatuses with or without conventional operating systems, for example LINUX, UNIX, WINDOWS, MAC OS, ANDROID, IOS or any other suitable conventional operating system.

Any combination of one or more computer readable medium(s) may be utilized. The computer readable medium may be a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

Generally, the program instructions can be executed on one processor or on several processors. In the case of multiple processors, they can be distributed over several different entities. Each processor could execute a portion of the instructions intended for that entity. Thus, when referring to a system or process involving multiple entities, the computer program or program instructions are understood to be adapted to be executed by a processor associated or related to the respective entity.

While the invention has been illustrated and described in detail in the drawings and foregoing description, such illustration and description are to be considered illustrative or exemplary and not restrictive; the invention is not limited to the disclosed examples.

## Claims

1. A method of generating a training dataset (300) for training a machine learning model for prediction of a specific property of a material, the method comprising:
repeatedly performing the following:
selecting (201) a hypothetical material from a parameter space, the parameter space being defined by a set of material parameters descriptive of materials;
using (203) a quantum computer (102) for numerically solving an electronic Schrödinger equation for a quantum system representing the selected hypothetical material;
using (205) the solution of the electronic Schrödinger equation for obtaining the specific property of the selected hypothetical material;
adding (207) an entry to the training dataset, the entry indicating the selected hypothetical material and a label, wherein the label indicates the obtained specific property.

2. The method of claim 1, wherein the specific property is a band gap.

3. The method of claim 1 or 2, the parameter space comprising points, each point having respective values of the set of material parameters, wherein the parameters space is defined using a bin packing problem such that the parameter space is uniformly filled by the points.

4. The method of any of the preceding claims, wherein the solution of the electronic Schrödinger equation comprises wave functions that define a Hilbert space, wherein the specific property is obtained by providing an observable having specific basis in the Hilbert space and by projecting the wave functions into the specific basis.

5. The method of any of the preceding claims, wherein the parameter space is defined by a Smooth Overlap of Atomic Positions (SOAP) descriptor.

6. The method of any of the preceding claims, the set of parameters being descriptive of chemical composition and atomic configuration of the material, wherein the set of material parameters comprise: nuclear charge, and orbital radius electronegativity.

7. The method of any of the preceding claims, further comprising using the training dataset to train the machine learning model to predict the specific property for input materials.

8. The method of claim 7, further comprising:
predicting a band gap of a hypothetical material using the trained machine learning model;
in response to determining that a color determined by the band gap fulfils a selection criterion, using the hypothetical material for creating an identity document.

9. A computer program comprising machine executable instructions, wherein execution of the machine executable instructions causes a computer system to perform at least the following:
repeatedly performing the following:
selecting a hypothetical material from a parameter space, the parameter space being defined by material parameters descriptive of materials;
using a quantum computer for numerically solving an electronic Schrödinger equation for a quantum system representing the selected material;
using the solution of the electronic Schrödinger equation for obtaining a specific property of the selected material;
adding an entry to the training dataset, the entry indicating the selected material and a label, wherein the label indicates the obtained specific property.

10. A computer system (100) of generating a training dataset for training a machine learning model for prediction of a specific property of a material, the computer system being configured for:
repeatedly performing the following:
selecting a hypothetical material from a parameter space, the parameter space being defined by material parameters descriptive of materials;
using a quantum computer for numerically solving an electronic Schrödinger equation for a quantum system representing the selected hypothetical material;
using the solution of the electronic Schrödinger equation for obtaining the specific property of the selected hypothetical material;
adding an entry to the training dataset, the entry indicating the selected hypothetical material and a label, wherein the label indicates the obtained specific property.

11. A method for band gap prediction, the method comprising:
receiving (501) a description of a desired material;
inputting (503) the description to a trained machine learning model, wherein the machine learning model is trained using training dataset of claim 1;
receiving (505) an output of the trained machine learning model indicating the band gap of the desired material.

12. The method of claim 11, further comprising,
determining whether the desired material has a desired color using the predicted band gap,
in case the desired material has the desired color, inputting the description of the desired material into a robotic system, the robotic system including automated chemical reactors, and material deposition techniques.

13. A computer program comprising machine executable instructions, wherein execution of the machine executable instructions causes a computer system to perform at least the following:
receiving a description of a desired material;
inputting the description to a trained machine learning model, wherein the machine learning model is trained using training dataset of claim 1;
receiving an output of the trained machine learning model indicating the band gap of the desired material.

14. A computer system for band gap prediction, the computer system being configured for:
receiving a description of a desired material;
inputting the description to a trained machine learning model, wherein the machine learning model is trained using training dataset of claim 1;
receiving an output of the trained machine learning model indicating the bandgap of the desired material.
